# EUROPEAN PATENT SPECIFICATION

(11) **EP 0 401 249 B1**
(45) Date of publication and mention of the grant of the patent: **04.05.1994**
(21) Application number: 89902345.1
(22) Date of filing: 10.02.1989
(51) Int. Cl.: A61K 37/00, C12N 11/08, C12N 1/04, C12N 15/00, C12N 1/20, A01N 63/00

(54) **ENCAPSULATED BACTERIUM**
EINGEKAPSELTE BAKTERIE
BACTERIE ENCAPSULEE

(30) Priority: 12.02.1988 US 155215
(43) Date of publication of application: 12.12.1990
(73) Proprietor: THE PRESIDENT AND FELLOWS OF HARVARD COLLEGE, Cambridge, MA 02114 (US)
(72) Inventor: ZOMER, Eliezer, Quincy, MA 02169 (US); SPIELMAN, Andrew, Newton Highlands, MA 02161 (US); PERRONE, Joseph, B., Hawthorne, NJ 07506 (US)
(74) Representative: Moon, Donald Keith
(86) International application number: US8900555
(87) International publication number: WO8907447

(56) References cited:
- WO-A-00/83267
- WO-A-00/97571
- WO-A-79/00155
- GB-A- 1 595 054
- US-A- 4 328 203
- US-A- 4 647 536
- US-A- 4 652 658
- US-A- 4 934 231
- CHEMICAL ABSTRACTS, vol. 109, no. 19, 7th November 1988, page 284, abstract no. 165747f, Columbus, Ohio, US

## Description

Bacillus thuringiensis israelensis (BTI) forms an intracellular insecticidal toxin, known as a parasporal crystal, during the process of spore formation. This toxin is taken up by insects, such as mosquitoes, and is broken down by the alkaline contents of the insect gut, where it causes disintegration of the gut and kills the insect, The insect toxin is commonly produced by fermenting BTI to form a cell mass, inducing sporulation of these cells, allowing toxin production and spore formation, and then allowing lysis of the mother cell to release the spore and parasporal crystal into the culture medium. The crystals and associated spores are then harvested and the insecticide is formulated and packaged. This insecticide is spread over the site. See, for example, Aizawa, "Microbial Control of Insect Pests", Ch. 15 in Advances in Agricultural Microbiology, pp. 397 et seq., Subba [Ed.] Oxford IBH (1982); Abstract of Lacey et al. J. Am. Mosq. Control. Ass. 1:493-497 (1985); Abstract of Novak et al. J. Am. Control Ass. 1:449-453 (1985).

In a first aspect, the invention features a bacterium containing an insecticidal toxin, wherein the bacterium is encapsulated by a non-living resinous protective material that is permeable to digestive enzymes of the target insect at highly alkaline pH (e.g. pH above 9.0). Encapsulated means completely surrounded by the polymeric material and thus protected at pH levels experienced in the environment (e.g. pH below 8.5) from environmental factors including heat, humidity and solutions, such as carbonic acid formed from rain water. "Protective" means that the material significantly retards degradation of the encapsulated bacterium under conditions experienced in the agricultural environment of use.

In preferred embodiments, the toxin is parasporal crystal toxin and the bacterium is Bacillus thuringiensis; the polymer is stable in an agricultural environment, for example in soil or on the leaves of plants; specifically, the polymer is heat stable (up to at least about 49°C (120°F)), and is not rapidly degraded by water (pH below 8.5), ultraviolet radiation and other conditions experienced in the agricultural environment. Moreover, the polymer protects the sporangia against other environmental degradation such as microbial or enzymatic degradation. The polymer need not be significantly degraded in the insect gut, so long as it is readily permeated by insect gut fluid (e.g., above pH 10) which lyses the sporangia. The polymer should be permeable to the toxin under those conditions to release it to the insect gut. The toxin is produced by growing the bacterium to stage III of sporulation. The polymer can be nonionic, polyanionic, or polycationic. A suitable nonionic polymer is dextran, starch or gelatin; a suitable polyanionic polymer is alginate or carageenan, and a suitable polycationic polymer is chitosan or polyoxyethylene bis amine. Other preferable polymer characteristics are: (a) the polymer is cross-linked, most preferably with formaldehyde; glutaraldehyde; DMA (dimethyl adipimidate 2HCl as disclosed in Waterman et al. (1975) Biochem. Biphys. Res. Commun. 63:580-587); DMS (dimethyl suberimidate 2HCl as disclosed in Packman et al. (1982) Biochemistry 21:5171-5175); BSOCOES (bis[2-(succinimido-oxycarbonyloxy)ethyl] sulfone as disclosed in Zarling et al. (1980) J. Immunol. 124:913-920; DST (disuccinimidyl tartarate as disclosed in Bragg et al. (1980) Eur. J. Biochem. 106:495-503); sulfo-BSOCOES (Zarling et al.); sulfo-DST (Bragg et al.); polyphosphate ions; or metal ions (e.g. Ca²⁺); and (b) the polymer contains an additive such as a sunscreening chromophore, a flavouring agent, or an attractant such as a pheromone.

In a second aspect, the invention features an aggregate of bacterial cells containing the bacterium described above. Preferably the aggregate has a diameter of 10 to 100 µm, most preferably of 20-50 microns.

In the third aspect, the invention features a method for producing an encapsulated insecticide. The method includes growing a bacterium having a gene encoding for an insect toxin to form the insecticide, and encapsulating the bacterium in a polymer.

In preferred embodiments, the bacterium is grown to stage III of sporulation; and the encapsulating includes coating the outer membrane of the bacterium.

The invention takes advantage of characteristics of the microbial cell wall to attract cellular polymers, and thus be encapsulated. Stabilization of these encapsulated preparations, by chemical cross-linking, produces a highly stable product suitable for control of insects and is important both for public health and for agriculture. In preferred aspects, the toxin is a stomach toxin, the microencapsulation enhances ingestion by the target insect, and also permits release and activation of the toxin by digestive enzymes operating within the alkaline medium of the gut of the target insect. In a particularly preferred embodiment, using, e.g., Bacillus thuringiensis, the process maintains the cells as intact sporanigia.

The encapsulated toxins are highly stable under field conditions and thus need be applied less frequently than previous insecticides, reducing costs in their use. Further, the process for manufacturing these insecticides is relatively inexpensive. Since it is preferred that the toxin remains within a bacterium, the fermentation process is shortened. For example, sporulating bacteria need only be grown until production has been completed, i.e., stage III of sporulation. At this point, the cells are coated directly with encapsulating polymer and can be sold, distributed and applied to agricultural products in this state. The encapsulated toxin has a considerably longer half-life compared to nonencapsulated toxin under natural and simulated field conditions. Further, since the cells can be aggregated by cross-linking of the polymer, the diameter of the aggregate can be chosen so that it is optimal for the feeding requirements of the target insect. For example, with mosquitoes (some of which are filter feeders), an increasing toxicity is observed when particles are of 25-100 µm in diameter, for smaller larvae a size of 10-20 µm is better.

An added feature of this invention is the opportunity and ability to incorporate sunscreening chromophores food additives, dyes and pheromones into the polymers. The chromophores protect the encapsulated toxin from UV irradiation, thus enhancing its stability. The food additives and pheromones enhance the chance that an insect will eat the toxin.

Other features and advantages of the invention will be apparent from the following description of the preferred embodiments, and from the claims.

### Toxins

Toxins suitable in this invention include insecticidal toxins. Suitable toxins include those which are commonly used; and in some instances they are unstable in the normal agricultural environment of use. These toxins include the parasporal crystal toxin of BTI, or B. thurgiensis entomotsides, and related such toxins. See generally, Aizawa, cited above, at p. 400. The toxins may be produced by commonly used procedures, or by a modified procedure, such as that described below. The toxins may also be produced by expression of toxin-encoding genes within other bacteria, such as E. coli or B. subtilis, using standard techniques as disclosed by McLean and Whitely, J. Bacteriol. 169(3): 1017-1023 (1987); and by Ward et al., J. Mol. Biol. 191:13-22 (1986). Preferably the toxins remain within these bacteria during the encapsulation process.

### Encapsulating Polymer

Polymers suitable for encapsulating the above toxins include a variety of nonionic, polyanionic, and polycationic polymers. Examples of such polymers are dextrans, such as starch, proteins such as gelatin, alginate or carogeenan and chitosan and polyoxyethylene bis amine. These polymers are generally biopolymers which are extensively biodegradable; however, they are preferably sufficiently resistant to a wide variety of normal agricultural-type conditions, to protect the toxin for a period that allows it to be ingested and substantially reduces the time between applications. The polymer protects against bacterial or enzymatic degradation of the sporangia. The polymers are highly porous and accessable to the alkaline condition and digestive enzymes, such as in the gut of an insect.

### Example

In this example, the sporangia containing the parasporal crystal toxin of BTI is encapsulated by one of a variety of encapsulating polymers.

This example is not limiting to the invention; those skilled in the art will realize that the above described toxins and encapsulating polymers can be substituted in this example.

Cells of BTI are grown in submerged culture for about 12-18 hours at 32°C using baffled shaker flasks at 100 rpm. Alternatively, the cells are grown in a fermentor with 0.8 vols. air per vol. medium, until they reach phase III of sporulation. This stage is determined by standard procedure, e.g., microscopically or by monitoring the metabolic heat output of the bacteria. At this stage of the fermentation the temperature is reduced from 32°C to 22°C, or the pH is lowered from 7 to 6 by addition of sulphuric acid. These environmental changes help to delay lysis of the bacterium by more than 24 hours. This delay allows encapsulation of the bacterium to proceed without the toxin crystals being released from the sporulating mother cells or sporangia.

The sporangia are encapsulated in one of the three types of polymers described below. (The cells may be concentrated prior to this process by simply allowing them to settle, and decanting off about 80% of the liquid remaining above the precipitate. Alternatively, the cells may remain in suspension.)
a) A nonionic polymer such as soluble starch is added to the culture media, followed by an organic solvent such as ethanol or acetone up to 50% of volume. These solvents act as coacervating agents and cause the coated cells to be precipated out of the culture medium. Generally the amount of starch added is about equal to the amount of cells in the media and its concentration is about 0.5-5% by weight. The polymer also can be precipitated out of the solvent by a rapid temperature shift.
b) A polycationic polymer such as chitosan is added to the medium (at about 0.4-4% by weight). The chitosan has been acidified with acetic acid (0.2-0.5 M) to a pH less than about 5. The negatively charged sporangia walls attract the soluble polymer. The polymers are then stabilized with polyphosphate ions (0.1-0.2 M).
c) An anionic polymer, such as alginate, is added to the medium (at about 0.4-4% by weight). These polymers are attracted to the cells. Calcium salts (0.1-0.2 M), are then added to cross-link the polymer and to produce cells which have multiple layers of polymer.

Colour attractants such as dyes or U.V. screening compounds, e.g., para amino benzoic acid, are added at this stage.

The coating process takes only a few minutes, but the mixture may be left for 1-2 hours. The coated sporangia, along with additives, are now chemically cross-linked to stablize the final product. Cross-linking substances (at about 0.1-0.01% by weight) such as aldehydes, e.g., formaldehyde or glutaraldehyde, bifunctional amidoesters, such as DMA or DMS, bifunctional N-hydroxy succinimide esters such as BSOCOES or DST, and others listed in the Pierce Handbook and Catalog (1986-7, pp. 311-340) are suitable. The most suitable such cross-linking agents are base reversible and thus cleaved within the gut of the targeted insect. See Zarling et al. cited above. These cross-linking substances thus allow enhanced release of the toxin within the gut, and eventual activation of the toxin.

The final product consists of a sporangium encapsulated by cross-linked polymer, and can be spray dried or sold as an emulsion, and applied directly to the target plants. Spray drying is performed by standard procedures using an atomizer and spraying the mixture into a calcuim or polyphosphate solution, as appropriate. Alternatively, the mixture can be preserved by addition of benzoic or sorbic acids, which are commonly used as food preservatives at 0.1%. Such solutions and dried products are stable for several months and even years. They are applied by standard techniques to plants and fields, but the amount of insecticide used is less than with prior formulations because lower frequency of application is necessary.

## Claims

1. A population of encapsulated bacteria, containing an insecticidal toxin, and being encapsulated by a non-living, resinous, protective non-ionic, polyanionic or polycationic polymer that is permeable to insect digestive enzymes at alkaline pH encountered in the insect gut, said bacteria being sporangia

2. The bacteria of claim 1 wherein said toxin is a parasporal crystal toxin.

3. The bacteria of claim 2 wherein said bacteria is Bacillus thuringiensis.

4. The bacteria of any of claims 1 to 3 wherein said polymer is stable in soil or on leaves

5. The bacteria of any of claims 1 to 4 wherein said polymer is porous to insect gut digestive juices.

6. The bacteria of any of claims 1 to 5 wherein said polymer is substantially impermeable to biological agents capable of degrading said bacterium.

7. The bacteria of any of claims 1 to 6 wherein said bacterium is produced harvesting a colony at stage III of sporulation.

8. The bacteria of any of claims 1 to 8 wherein said polymer is selected from the group consisting of dextran, starch, gelatin, alginate, carrageenan, chitosan, and polyoxyethylene bis amine.

9. The bacteria of any of claims 1 to 8 wherein said polymer is cross-linked.

10. The bacteria of claim 9 wherein said polymer is cross-linked with formaldehyde, glutaraldehyde, DMA, DMS, BSOCOES, DST, polyphosphate ions or metals.

11. The bacteria of any of claims 1 to 10 wherein said polymer comprises an additive selected from the group consisting of sunscreening chromophores, phagostimulants, and insect attractants.

12. An aggregate of encapsulated bacterial cells comprising the bacterium of any of claims 1 to 11.

13. The aggregate of claim 12 having a diameter of 10-100 µm.

14. The aggregate of claim 12 having a diameter of 20-50 µm.

15. A method for producing an insecticide, comprising the steps of: growing a population of bacterial sporangia , said bacteria having a gene encoding for an insect toxin to form said toxin, and encapsulating said bacteria in a non-ionic, polyanionic or polycationic polymer.

16. The method of claim 15 wherein said growing comprises causing said bacterial to enter stage III of sporulation.

17. The method of claims 15 or 16 wherein said encapsulating comprises coating the outer membrane of said bacteria with said polymer.

18. The method of claim 16 wherein said bacteria is coated with a polymer that is porous to insect gut digestive juices and is impermeable to biological agents capable of degrading said bacteria

## Patentansprüche

1. Population von eingekapselten Bakerien, die ein Insektengift enthalten und die durch ein nicht lebendes, harzartiges, schützendes und nicht-ionisches oder poly-anionisches oder poly-kationisches Polymer eingekapselt sind, das für Verdauungsenzyme von Insekten bei alkalischen pH-Werten, die im Insektendarm auftreten, durchläßig ist, wobei das Bakterium Sporangia ist.

2. Bakterien nach Anspruch 1, wobei das Toxin ein parasporales kristallines Toxin ist.

3. Bakterien nach Anspruch 2, wobei das Bakterium Bazillus thuringiensis ist.

4. Bakterien nach einem der Ansprüche 1 bis 3, wobei das Polymer in Erde oder auf Blättern stabil ist.

5. Bakterien nach einem der Ansprüche 1 bis 4, wobei das Polymer für Verdauungssäfte des Insektendarms porös ist.

6. Bakterien nach einem der Ansprüche 1 bis 5, wobei das Polymer für biologische Agenzien, die in der Lage sind, das Bakterium zu beeinträchtigen, im wesentlichen undurchlässig ist.

7. Bakterien nach einem der Ansprüche 1 bis 6, wobei das Bakterium durch Ernten einer Kolonie in Stufe III der Sporulation erzeugt wird.

8. Bakterien nach einem der Ansprüche 1 bis 7, wobei das Polymer aus der Gruppe ausgewählt wird, die Dextran, Stärke, Gelatine, Alginat, Carrageenan, Chitosan und Polyoxiethylenbisamin umfaßt.

9. Bakterien nach einem der Ansprüche 1 bis 8, wobei das Polymer kreuzvernetzt ist.

10. Bakterien nach einem der Ansprüche 1 bis 9, wobei das Polymer mit Formaldehyd, Glutaraldenyd, DMA, DMS, BSOCOES, DST, Polyphosphat-Ionen oder Metallen kreuzvernetzt ist.

11. Bakterien nach einem der Ansprüche 1 bis 10, wobei das Polymer ein Additiv enthält, das aus der Gruppe ausgewählt ist, die Sonnenlicht abschirmende Chromophoren, Phagostimulanzien und Insektenlockstoffen umfaßt.

12. Aggregat von eingekapselten Bakterienzellen, welches die Bakterien nach einem der Ansprüche 1 bis 11 umfaßt.

13. Aggregat nach Anspruch 12, das einen Durchmesser von 10 bis 100 µm aufweist.

14. Aggregat nach Anspruch 12, das einen Durchmesser von 20 bis 50 µm aufweist.

15. Verfahren, um ein Insektizid zu produzieren, das die Schritte umfaßt: Wachsenlassen einer Population von Sporangia-Bakterien, wobei die Bakterien ein Gen aufweisen, das für ein Insektengift codiert, um das Toxin zu bilden, und Einkapselung der Bakterien in ein nicht-ionisches, poly-anionisches oder poly-kationisches Polymer.

16. Verfahren nach Anspruch 15, wobei das Wachsenlassen beinhaltet, die Bakterien in Stufe III der Sporulation eintretn zu lassen.

17. Verfahren nach Anspruch 15 oder 16, wobei die Einkapselung die Beschichtung der äußeren Membran der Bakterien mit dem Polymer umfaßt.

18. Verfahren nach Anspruch 16, wobei die Bakterien mit einem Polymer beschichtet sind, das für Verdauungssäfte des Insektendarms porös ist und das für biologische Agenzien, die in der Lage sind, die Bakterien zu beeinträchtigen, undurchlässig ist.

## Revendications

1. Population de bactéries encapsulées contenant une toxine insecticide et étant encapsulées par un polymère de protection inerte, résineux, non ionique, polyanionique ou polycationique, lequel est perméable aux enzymes digestives de l'insecte aux pH alcalins rencontrés dans l'intestin de l'insecte, lesdites bactéries étant sous forme de sporanges.

2. Bactéries selon la revendication 1, ladite toxine étant une toxine cristalline des paraspores.

3. Bactéries selon la revendication 2, lesdites bactéries étant Bacillus thuringiensis.

4. Bactéries selon l'une quelconque des revendications 1 à 3, ledit polymère étant stable dans le sol ou sur les feuilles.

5. Bactéries selon l'une quelconque des revendications 1 à 4, ledit polymère étant poreux vis-à-vis des sucs digestifs intestinaux de l'insecte.

6. Bactéries selon l'une quelconque des revendications 1 à 5, ledit polymère étant essentiellement imperméable aux agents biologiques capables de dégrader ladite bactérie.

7. Bactéries selon l'une quelconque des revendications 1 à 6, lesdites bactéries étant produites en récoltant une colonie ayant atteint le stade III de la sporulation.

8. Bactéries selon l'une quelconque des revendications 1 à 8, ledit polymère étant choisi parmi le dextrane, l'amidon, la gélatine, l'alginate, le carragheen, le chitosane et la polyoxyéthylène bis amine.

9. Bactéries selon l'une quelconque des revendications 1 à 9, ledit polymère étant réticulé.

10. Bactéries selon la revendication 9, ledit polymère étant réticulé avec du formaldéhyde, du glutaraldéhyde, du DMA, du DMS, du BSOCOES, du DST, des ions polyphosphate ou des polyphosphates de métaux.

11. Bactéries selon l'une quelconque des revendications 1 à 10, ledit polymère comprenant un additif choisi parmi les chromophores faisant écran solaire, les appétents, et les appâts pour insectes.

12. Agrégat de cellules bactériennes encapsulées comprenant la bactérie selon l'une quelconque des revendications 1 à 11.

13. Agrégat selon la revendication 12 ayant un diamètre de 10 à 100 µm.

14. Agrégat selon la revendication 12 ayant un diamètre de 20 à 50 µm.

15. Procédé pour la préparation d'un insecticide comprenant les étapes consistant à cultiver une population de sporanges bactériens, lesdites bactéries ayant un gène codant pour la toxine de l'insecte pour former ladite toxine et à encapsuler lesdites bactéries dans un polymère non ionique, polyanionique ou polycationique.

16. Procédé selon la revendication 15 dans lequel ladite culture comprend l'action d'amener lesdites bactéries au stade III de la sporulation.

17. Procédé selon la revendication 15 ou 16 dans lequel l'action d'encapsuler comprend le revêtement de la membrane externe desdites bactéries avec ledit polymère.

18. Procédé selon la revendication 16 dans lequel lesdites bactéries sont revêtues avec un polymère poreux vis-à-vis des sucs digestifs intestinaux de l'insecte et imperméable aux agents biologiques capables de dégrader lesdites bactéries.
